(19) Europäisches Patentamt
European Patent Office
Office européen des brevets

(11) Veröffentlichungsnummer: **0 200 065**
**A1**

(12) # EUROPÄISCHE PATENTANMELDUNG

(21) Anmeldenummer: 86105085.4

(22) Anmeldetag: 14.04.86

(51) Int. Cl.⁴: C 07 D 251/46
C 07 D 251/42

(30) Priorität: 27.04.85 DE 3515287

(43) Veröffentlichungstag der Anmeldung: 05.11.86 Patentblatt 86/45

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Riebel, Hans-Jochem, Dr.
In der Beek 92
D-5600 Wuppertal 1(DE)

(54) **Verfahren zur Herstellung von 2-Cyanamino-4,6-dialkoxy-1,3,5-triazinen.**

(57) Die Erfindung betrifft ein neues Verfahren zur Herstellung von 2-Cyanamino-4,6-dialkoxy-1,3,5,-triazinen der allgemeinen Formel (I)

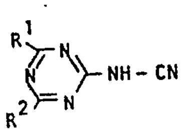

(I)

in welcher

$R^1$ und $R^2$ gleich oder verschieden sind und für Alkoxy stehen,

dadurch gekennzeichnet, daß man 4-Chlor-2-cyanamino-1,3,5-triazine der Formel (II)

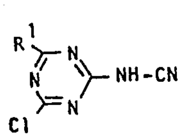

(II)

in welcher

$R^1$ die oben angegebene Bedeutung hat,

mit mindestens der zweifachen molaren Menge Alkoholat der Formel (III)

$R^2Me$ (III)

in welcher

$R^2$ die oben angegebenen Bedeutungen hat und

Me für ein Äquivalent eines Alkalimetallions steht,

in Gegenwart von Verdünnungsmitteln und in Gegenwart von Säuren umsetzt, und neue Zwischenprodukte zu ihrer Herstellung.

EP 0 200 065 A1

Croydon Printing Company Ltd.

0200065

BAYER AKTIENGESELLSCHAFT 5090 Leverkusen-Bayerwerk
Konzernverwaltung RP
Patentabteilung Bi/mü
IVa/ZP

Verfahren zur Herstellung von 2-Cyanamino-4,6-dialkoxy-1,3,5-triazinen

Die vorliegende Erfindung betrifft ein neues Verfahren zur Herstellung von 2-Cyanamino-4,6-dialkoxy-1,3,5-triazinen sowie neue Zwischenprodukte hierfür. Die Verfahrensprodukte sind zum Teil bekannt und können als Zwischenprodukte zur Herstellung von Herbiziden und Pflanzenwachstumsregulatoren verwendet werden.

Es ist bereits bekannt, daß 2-Cyanamino-1,3,5-triazine durch Umsetzung von Alkalimetall- oder Erdalkalimetall-Salzen von Cyanamid mit entsprechenden 2-Halogen-1,3,5-triazinen erhalten werden (vgl. z.B. DE-OS 3 334 455/EP-A- 121 082). Dieses Verfahren ist jedoch wegen des Fehlens geeigneter Ausgangsverbindungen bzw. wegen unbefriedigender Herstellungsmethoden hierfür nur sehr begrenzt anwendbar. Es besteht daher Bedarf an breiter anwendbaren Herstellungsverfahren für 2-Cyanamino-4,6-dialkoxy-1,3,5-triazine.

Es wurde nun gefunden, daß man 2-Cyanamino-2,6-dialkoxy-1,3,5-triazine der allgemeinen Formel (I)

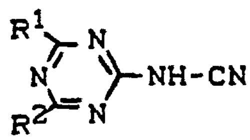

(I)

in welcher

$R^1$ und $R^2$ gleich oder verschieden sind und für Alkoxy stehen,

erhält, wenn man 4-Chlor-2-cyanamino-1,3,5-triazine der Formel (II)

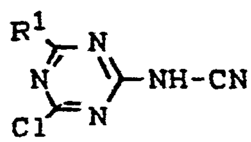

(II)

in welcher

$R^1$ die oben angegebenen Bedeutungen hat,

mit mindestens der zweifachen molaren Menge Alkoholat der Formel (III)

$$R^2Me \quad (III)$$

in welcher

$R^2$ die oben angegebenen Bedeutungen hat und

Me für ein Äquivalent eines Alkalimetallions steht,

in Gegenwart von Verdünnungsmitteln umsetzt, anschließend mit Wasser versetzt und ansäuert.

Überraschenderweise lassen sich mit Hilfe des erfindungsgemäßen Verfahrens die wertvollen Verbindungen der Formel (I) in glatter Reaktion und in hohen Ausbeuten auf einfache Weise über die neuen Zwischenprodukte der Formel (II) herstellen.

Bevorzugt werden mit Hilfe des erfindungsgemäßen Verfahrens die Verbindungen der Formel (I) hergestellt, in welcher

$R^1$ und $R^2$ gleich oder verschieden sind und für Alkoxy mit 1 bis 6 Kohlenstoffatomen stehen.

Besonders bevorzugt werden diejenigen Verbindung der Formel (I) hergestellt, in welcher

$R^1$ und $R^2$ gleich oder verschieden sind und für Methoxy, Ethoxy, n-Propoxy, i-Propoxy, n-Butoxy, i-Butoxy, sec.-Butoxy oder tert.-Butoxy stehen.

Ganz besonders bevorzugt werden diejenigen Verbindungen der Formel (I) hergestellt, in welcher

$R^1$ und $R^2$ gleich oder verschieden sind und für Methoxy, Ethoxy, n-Propoxy, i-Propoxy oder n-Butoxy stehen.

Verwendet man beispielsweise für das erfindungsgemäße Verfahren 4-Chlor-2-cyanamino-6-methoxy-1,3,5-triazin und Natriummethanolat als Ausgangsstoffe, so kann die Reaktion durch das folgende Formelschema wiedergegeben werden:

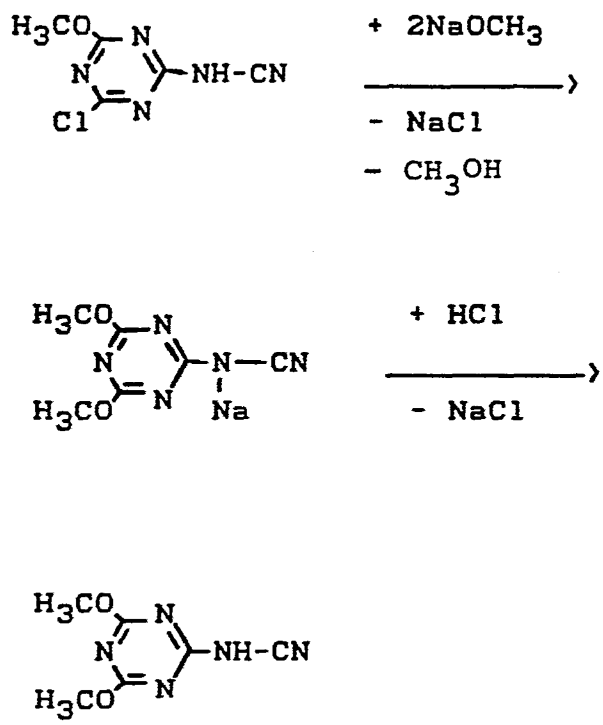


Die als Ausgangsstoffe für das erfindungsgemäße Verfahren zu verwendenden 4-Chlor-2-cyanamino-1,3,5-triazine sind durch die Formel (II) allgemein definiert. Bevorzugt sind diejenigen Verbindungen der Formel (II), in welcher

$R^1$ für Alkoxy mit 1 bis 6 Kohlenstoffatomen steht.

Besonders bevorzugt sind diejenigen Verbindungen der Formel (II), in welcher

$R^1$ für Methoxy, Ethoxy, n-Propoxy, i-Propoxy, n-Butoxy, i-Butoxy, sec.-Butoxy oder tert.-Butoxy steht.

Ganz besonders bevorzugt sind diejenigen Verbindungen der Formel (II), in welcher

$R^1$ für Methoxy, Ethoxy, n-Propoxy, i-Propoxy, oder n-Butoxy steht.

Die Verbindungen der Formel (II) sind neu. Man erhält die Verbindungen der Formel (II), wenn man 2,4-Dichlor-1,3,5-triazine der Formel (IV)

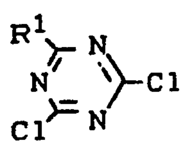 (IV)

in welcher

$R^1$ die oben angegebenen Bedeutungen hat,

mit Cyanamiden der Formel (V),

$$(R^3)_2N\text{-}CN \quad (V)$$

in welcher

$R^3$ für ein Äquivalent eines Alkali- oder Erdalkalimetallions steht,

in Gegenwart von Wasser und gegebenenfalls in Gegenwart eines organischen Verdünnungsmittels, wie z.B. Aceton oder Acetonitril, bei einem pH-Wert zwischen 8,5 und 9,5 und bei Temperaturen zwischen -5°C und +10°C zu den neuen Verbindungen der Formel (VI),

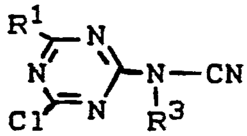

(VI)

in welcher

$R^1$ und $R^3$ die oben angegebenen Bedeutungen haben,

umsetzt und diese Verbindungen der Formel (VI) anschließend, gegebenenfalls nach ihrer Isolierung, in Gegenwart von Wasser und in Gegenwart von Säuren, wie z.B. Salzsäure, bei Temperaturen zwischen -10°C und +20°C umsetzt.

Als Beispiele für die Ausgangsstoffe der Formel (II) bzw. (VI) seien beispielsweise genannt:
6-Methoxy-, 6-Ethoxy-, 6-n-Propoxy-, 6-i-Propoxy-, 6-n-Butoxy-, 6-i-Butoxy-, 6-sec.-Butoxy- und 6-tert.-Butoxy-4-chlor-2-cyanamino-1,3,5-triazin bzw. die entsprechenden Natrium-, Kalium- und Calciumsalze.

Die als Ausgangsstoffe für die Herstellung der Verbindungen der Formel (II) bzw. (VI) zu verwendenden 2,4-Dichlor-1,3,5-triazine sind durch die Formel (IV) allgemein definiert. In dieser Formel steht $R^1$ bevorzugt für diejenigen Reste, welche oben im Rahmen der Substituentendefinition der Formel (II) vorzugsweise bzw. als insbesondere bevorzugt angegeben sind.

Als Beispiele für die Verbindungen der Formel (IV) seien genannt:
6-Methoxy-, 6-Ethoxy-, 6-n-Propoxy-, 6-i-Propoxy-, 6-n-Butoxy-, 6-i-Butoxy-, 6-sec.-Butoxy- und 6-tert.-Butoxy-2,4-dichlor-1,3,5-triazin.

Die Verbindungen der Formel (IV) sind bekannt oder lassen sich nach bekannten Verfahren in analoger Weise herstellen.

Die weiterhin als Ausgangsstoffe für die Herstellung der Verbindungen der Formel (II) bzw. (VI) zu verwendenden Cyanamide sind durch die Formel (V) allgemein definiert. In dieser Formel steht $R^3$ vorzugsweise für ein Äquivalent eines Natrium-, Kalium- oder Calciumions.

Als Beispiele für die Verbindungen der Formel (V) seien beispielsweise genannt:
Di-Natrium-cyanamid, Di-Kalium-cyanamid und Calciumcyanamid.

Die Verbindungen der Formel (V) sind allgemein bekannte Verbindungen der organischen Chemie.

Die weiterhin als Ausgangsstoffe für das erfindungsgemäße Verfahren zu verwendenden Alkoholate sind durch die Formel (III) allgemein definiert. In dieser Formel hat $R^2$ vorzugsweise diejenigen Bedeutungen, welche oben im Rahmen der Substituentendefinition der Formel (I) vorzugsweise bzw. als insbesondere bevorzugt angegeben sind. Me steht in dieser Formel vorzugsweise für ein Natrium- oder Kaliumion.

Als Beispiele für die Verbindungen der Formel (III) seien beispielsweise genannt:
Natrium-methylat, -ethylat, -n-propylat, -i-propylat, -n-butylat, -i-butylat, -sec.-butylat und -tert.-butylat und die entsprechenden Kalium-Derivate.

Die Verbindungen der Formel (III) sind allgemein bekannte Verbindungen der organischen Chemie.

Das erfindungsgemäße Verfahren wird in Gegenwart von Verdünnungsmitteln durchgeführt.

Hierzu gehören insbesondere aliphatische und aromatische Kohlenwasserstoffe, wie Pentan, Hexan, Heptan, Cyclohexan, Petrolether, Benzin, Ligroin, Benzol, Toluol, Xylol; Alkohole, wie Methanol, Ethanol, n-Propanol, i-Propanol, n-Butanol, i-Butanol, sec.-Butanol und tert.-Butanol. Bevorzugt werden die entsprechenden Alkohole der eingesetzten Alkoholate der Formel (III) verwendet.

Das erfindungsgemäße Verfahren wird im allgemeinen bei Temperaturen zwischen 0°C und 50°C, vorzugsweise zwischen 0°C und 40°C durchgeführt. Die Umsetzungen werden im allgemeinen bei Normaldruck durchgeführt.

Bei der Durchführung des erfindungsgemäßen Verfahrens setzt man auf ein Mol der Verbindung der Formel (II) 2,0 bis 2,5 Mol, vorzugsweise 2,0 bis 2,3 Mol Alkoholat

der Formel (III) ein. Die Aufarbeitung der Verbindungen der Formel (I) geschieht nach üblichen Methoden. Nach beendeter Zugabe der Ausgangsstoffe wird noch kurze Zeit oder auch mehrere Stunden bei 15°C bis 25°C nachgerührt. Anschließend wird eingeengt und der Rückstand mit Wasser versetzt und mit einer Mineralsäure, wie z.B. Salzsäure angesäuert. Die Verbindungen der Formel (I) fallen im allgemeinen in kristalliner Form an.

Die nach den erfindungsgemäßen Verfahren herzustellenden 2-Cyanamino-4,6-dialkoxy-1,3,5-triazine können als Zwischenprodukte für die Herstellung von Guanidin-Derivaten, die als Herbizide und Pflanzenwuchsregulatoren wirksam sind, eingesetzt werden (vgl. EP-OS 121 082).

0200065

## Herstellungsbeispiele

### Beispiel 1

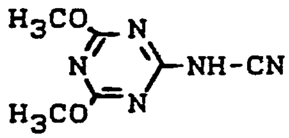

Zu einer Suspension von 6 g (0,032 Mol) 4-Chlor-2-cyanamino-6-methoxy-1,3,5-triazin in 50 ml Methanol tropft man bei 20°C bis 30°C eine Lösung von 1,6 g (0,069 Mol) Natrium in 20 ml Methanol. Anschließend wird bei 20°C 15 Stunden nachgerührt. Das Lösungsmittel wird abdestilliert, der Rückstand in 30 ml Wasser gelöst und mit Salzsäure angesäuert. Die entstandenen Kristalle werden abgesaugt und getrocknet.

Man erhält 5,9 g (92 % der Theorie) 2-Cyanamino-4,6-dimethoxy-1,3,5-triazin. Das Produkt wird durch $^{1}H$-NMR-Spektren charakterisiert.

Ausgangsprodukte der Formel (II)

Beispiel (II-1)

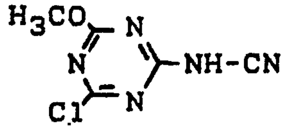

Eine Lösung von 9 g (0,043 Mol) 4-Chlor-2-cyanamino-6-methoxy-1,3,5-triazin-Natriumsalz in 90 ml Wasser wird mit Salzsäure angesäuert und die entstandenen Kristalle werden anschließend abgesaugt.

Man erhält 6,7 g (84 % der Theorie) 4-Chlor-2-cyanamino-6-methoxy-1,3,5-triazin vom Schmelzpunkt >260°C. Das Produkt wird durch $^{1}H$-NMR-Spektren charakterisiert.

Ausgangsprodukte der Formel (VI)

Beispiel (VI-1)

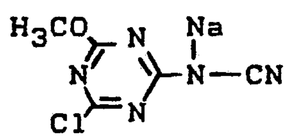

Zu einer Suspension von 36 g (0,2 Mol) 2,4-Dichlor-6-methoxy-1,3,5-triazin in 200 ml Eiswasser und 20 ml Aceton wird bei einer Temperatur von 0°C bis 5°C eine Lösung von 17,5 g Dinatriumcyanamid in 100 ml Wasser in der Weise zugetropft, daß ein pH-Wert von 9,5 nicht überschritten wird. Anschließend wird mit 40 g Natriumchlorid versetzt und 4 Stunden bei 20°C nachgerührt.

Nach dem Absaugen und Trocknen erhält man 36,9 g (89 % der Theorie) 4-Chlor-2-cyanamino-6-methoxy-1,3,5-triazin-Natriumsalz vom Schmelzpunkt 220°C (Zers.).

## Patentansprüche

1) Verfahren zur Herstellung von 2-Cyanamino-4,6-dialkoxy-1,3,5-triazinen der allgemeinen Formel (I)

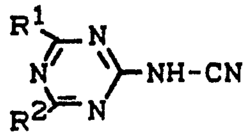

(I)

in welcher

$R^1$ und $R^2$ gleich oder verschieden sind und für Alkoxy stehen,

dadurch gekennzeichnet, daß man 4-Chlor-2-cyanamino-1,3,5-triazine der Formel (II)

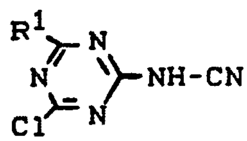

(II)

in welcher

$R^1$ die oben angegebene Bedeutung hat,

mit mindestens der zweifachen molaren Menge Alkoholat der Formel (III)

$R^2Me$ (III)

in welcher

$R^2$ die oben angegebene Bedeutung hat und

Me für ein Äquivalent eines Alkalimetallions steht,

in Gegenwart von Verdünnungsmitteln umsetzt, anschließend mit Wasser versetzt und ansäuert.

2) 4-Chlor-2-cyanamino-1,3,5-triazine der Formel (II)

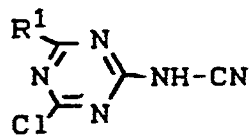

(II)

in welcher

$R^1$ für Alkoxy steht.

3) Verfahren zur Herstellung von 4-Chlor-2-cyanamino-1,3,5-triazinen der Formel (II), gemäß Anspruch 2, dadurch gekennzeichnet, daß man 2,4-Dichlor-1,3,5-triazine der Formel (IV)

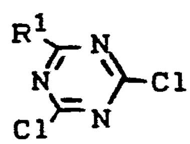

(IV)

in welcher

$R^1$ die oben angegebene Bedeutung hat,

mit Cyanamiden der Formel (V)

$$(R^3)_2N\text{-}CN \quad (V)$$

in welcher

$R^3$ für ein Äquivalent eines Alkali- oder Erdalkalimetallions steht,

in Gegenwart von Wasser und gegebenenfalls in Gegenwart eines organischen Verdünnungsmittels, bei einem pH-Wert zwischen 8,5 und 9,5 und bei Temperaturen zwischen -5°C und +10°C zu den Verbindungen der Formel (VI)

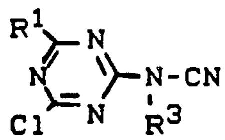 (VI)

in welcher

$R^1$ und $R^3$ die oben angegebenen Bedeutungen haben,

umsetzt und diese Verbindungen der Formel (VI) anschließend, gegebenenfalls nach ihrer Isolierung, in Gegenwart von Wasser und in Gegenwart von Säuren, wie z.B. Salzsäure, bei Temperaturen zwischen -10°C und +20°C umsetzt.

4) Verbindungen der Formel (VI)

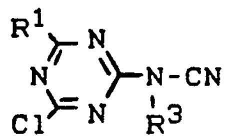 (VI)

in welcher

$R^1$ für Alkoxy steht und

$R^3$ für ein Äquivalent eines Alkali- oder Erdalkalimetallions steht.

0200065

Europäisches Patentamt

# EUROPÄISCHER RECHERCHENBERICHT

Nummer der Anmeldung

EP 86105085.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | GB - A - 1 052 567 (LONZA)<br>* Anspruch 1; Beispiele 1,2 *<br>-- | 1,2,4 | C 07 D 251/46<br>C 07 D 251/42 |
| A | CHEMICAL ABSTRACTS, Band 94, Nr. 19, 11 Mai 1981, Columbus, Ohio, USA<br>DOVLATYAN, V.V.; KHACHATRYAN, L.A.; AMBARTSUMYAN, E.N. "Action of acid chlorides, hydrochloric acid, and carbon dioxide on cyanamino-s-triazine salts"<br>Seite 656, Spalte 2, Zusammenfassung-Nr. 156 874q<br>& Arm. Khim. Zh. 1980, 33(9), 755-8<br>---- | 1 | |
| | | | RECHERCHIERTE SACHGEBIETE (Int. Cl.4) |
| | | | C 07 D 251/00 |

Der vorliegende Recherchenbericht wurde für alle Patentansprüche erstellt.

| Recherchenort | Abschlußdatum der Recherche | Prüfer |
|---|---|---|
| WIEN | 16-07-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument